# EUROPEAN PATENT APPLICATION

(11) **EP 2 916 248 A1**
(43) Date of publication of application: **09.09.2015**
(21) Application number: 14157428.5
(22) Date of filing: 03.03.2014
(51) Int. Cl.: G06F 19/00

(54) **Interactive medication prescription input tool**

(71) Applicant: Agfa HealthCare, 53227 Bonn (DE)
(72) Inventor: Mouluquet, Laurent, 2640 Mortsel (BE); Alard, Yohan, 2640 Mortsel (BE); Brun, Jerome, 2640 Mortsel (BE); Hickel, Bruno, 2640 Mortsel (BE)
(74) Representative: Verbrugghe, Anne Marie L.

(57) **Abstract**

A computer-implemented method for entering a medication prescription for a patient, comprising the steps of:
- entering medical information related to the medication prescription in an input module, and storing said information in a memory
- interpreting and processing the stored medical information in a processing module
- generating a medical prescription time-line chart characterized in that when the user interacts with graphical elements on the time-line chart, the medical information is updated in memory.

## Description

### FIELD OF THE INVENTION

The present invention relates generally to the management of medication prescriptions by means of charts or time-lines which are being created and maintained for an in-patient setting in a hospital.

### BACKGROUND OF THE INVENTION

The prescription and administration of a medicine is a common, but important, clinical procedure. It is the manner in which a medicine is administered that will determine to some extent whether or not the patient gains any clinical benefit, and whether they suffer any adverse effect from their medicines.

In a hospital in-patient setting, it is a complex aspect of the daily occupation for the staff to maintain overview on the different treatment schedules of the different patients. In the prior art, written templates have been used to create and maintain these medical charts for the patients during their treatment in the hospital. These written charts are used to keep track of the prescriptions and the delivery of the medication to the patient. The doctor will instruct the staff by means of a written (and signed) instruction about a prescription to be delivered to the patient.

However, these written charts are not dynamic and are not very easy to adjust or correct. It has been described in the prior art that nowadays these charts are replaced by electronic equivalents which are processed, stored and displayed in so-called medication management systems, generally known as part of a hospital information system.

Often these medical charts combine the prescription and treatment delivery records with other measurements (blood pressure, temperature), as well as other observations performed by the staff on the patient. These different records are often combined as separate pages to the bedside patient record, or are -in the case of an implementation in a hospital information system- integrated into a common database which then allows to extract and display the different records for a particular patient on an integrated chart.

A drug prescription prescribed by the doctor may constitute of different parameters like the route of administration, daily frequency of dosage, the strength, dosage form, the dosage application type (continuous, discontinuous (each can be prescribed with or without a duration)) and other parameters, which all need to be interpreted correctly by the caretaker. This mental activity is prone to human error, and induces a risk for faulty administration of medication to the patient.

It will be beneficial that the prescription can be recorded and presented to the caretaker (and prescribing physician) in a unified format, and that the prescription is presented in an unambiguous format which allows quick verification of the validity of the prescription which will be administered to the patient.

It is also desirable that the prescription can be modified or updated when necessary in an intuitive way.

### SUMMARY OF THE INVENTION

The present invention provides a computer-implemented method for entering a medication prescription for a patient.

The method comprises:
- entering medical information related to the medication prescription in an input module, and storing said information in a memory
- interpreting and processing the stored medical information in a processing module
- generating a medical prescription time-line chart characterized in that when the user interacts with graphical elements on the time-line chart, the medical information is updated in memory.

The above-described aspects are solved by a method as set out in claim 1.

The present invention can be implemented as a computer program product adapted to carry out the steps set out in the description. The computer executable program code adapted to carry out the steps set out in the description can be stored on a computer readable medium.

In the context of this invention, an input module for entering medical information should be understood as the data entry screen or set of screens of a medication management system, which allow inputting data in all required input fields for a complete prescription record to be created and stored in the computer memory (in the prescription database), where the actual data input is made through standard computer input devices such as a keyboard, a mouse, a touch screen, a barcode scanner, or other devices. A data entry screen in the context of this invention, is a combination of input fields shown together in the context of the entry of a complete medication prescription. Input fields are computer display elements which allow entry of input data, such as data entry widgets (text boxes, sliders, dropdown boxes, selection boxes, ...).

A prescription for a medication is characterised and defined by a number of parameters: the route of administration, the dosage application type (continuous or discontinuous), the dosage regime (frequency of applications) and the dosage itself (for instance: mg, drops, # of pills/capsules/..., or a measure which is expressed with a denominator, for instance: mg/kg bodymass,...)

The route of administration is the path by which a drug is taken into the body, this can be oral (sublingual), suppository, inhalation, topical or parenteral (intravenous, intramuscular, subcutaneous). Depending on the route of administration, dosage forms come in several types. These include many kinds of liquid, solid, and semisolid dosage forms. Common dosage forms include pills, tablets, or capsules, drink or syrup, and natural or herbal form such as plant or food of sorts, among many others. Notably, the route of administration for drug delivery is dependent on the dosage form of the substance in question. A liquid dosage form is the liquid form of a dose of a chemical compound used as a drug or medication intended for administration or consumption.

In the context of this invention, a time-line chart is a way of displaying a list of dosage events composing the prescription for a 24 hour period in chronological order. Notably, the individual dosage events are shown as individual marks on the time-line represented by a pictogram which typically represents a single dosage event, and which depicts the dosage form of the prescribed drug dosage event (the pictogram can be, but is not limited to, for instance an icon of a pill, a gel, an injection, a drop, a bar representing a an application over some time.) Other pictograms may be shown on the time-line indicating other events besides dosage events, such as the start-time of the medication treatment schedule.

The processing module may include dedicated hardware, software and/or firmware for performing information processing. The processing module can be connected to a memory, and can be capable of reading in data from the said memory and from input devices connected to the processor (such as a touch screen, a keyboard, a mouse, a bar code scanner, ...). The processor can also be connected to output devices, such as a printer, a computer screen or monitor, an external memory for rendering or storing the prescription time-line chart.

Specific examples and preferred embodiments are set out in the dependent claims.

In the context of this invention, the concept of "interactivity" applied to the prescription timeline means that certain graphically represented elements (such as the dosage events) can be manipulated by means of "dragging", "expanding" or "shrinking" them on the prescription timeline and serve as intuitive input from the users to make changes to the medication prescription.

The patient may be a human patient or an animal.

The visual format of the prescription time-line is an enhancement over the prior art as it allows immediate validation of the prescription as made by the caretaker who entered the prescription in the system.

The interactivity of the prescription time-line is an enhancement over the prior art as it allows immediate modification of the prescription in an intuitive and efficient way for the user.

### BRIEF DESCRIPTION OF THE DRAWINGS

Fig. 1 shows a flowchart illustrating a method for entering a medication prescription for a patient, consisting of the input form [100] module, the processing module [200] and the interactive timeline chart [300].
Fig 2. shows a similar flowchart as in Fig. 1 except for that the functioning of the processing module is explained in more detail.
Different embodiments of possible validation processes are depicted with their potential effects on the input form module, or the prescription timeline chart.

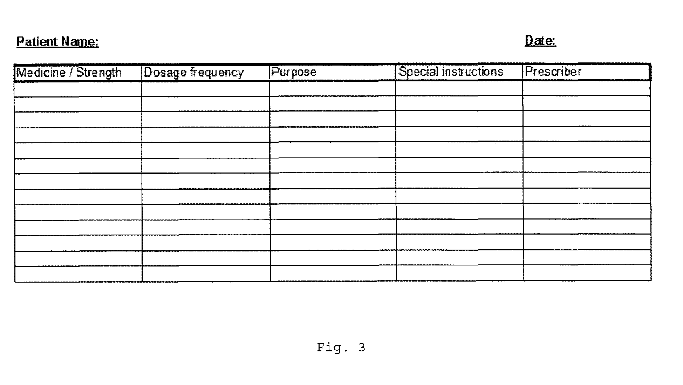

Fig 3. is a sample written template described in the prior art, to create and maintain medical charts for the patients during their treatment in the hospital.

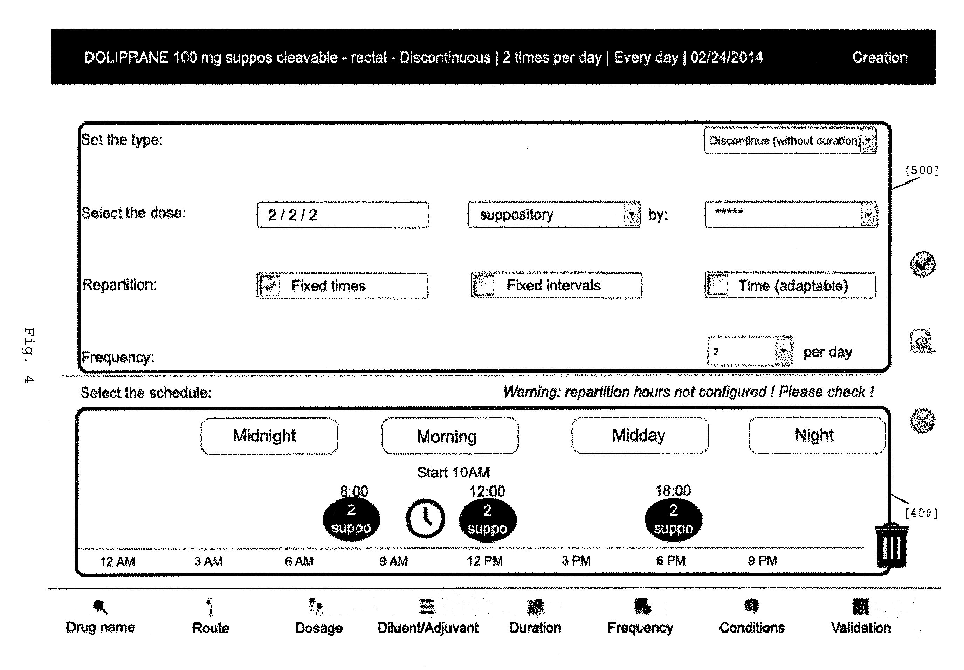

Fig 4. is an embodiment of the invention as implemented on a computer system showing an embodiment of the input form module [400] and an embodiment of the interactive timeline chart [500].

### DETAILED DESCRIPTION OF THE INVENTION

In the following detailed description, reference is made in sufficient detail to the above referenced drawings, allowing those skilled in the art to practice the embodiments explained below.

Embodiments of the present invention provide a method for entering and processing a medication prescription for a patient in a medical information management system. The medical information management system is typically implemented on a computer system comprising a means for inputting data (such as a keyboard, a touch screen, a computer mouse, a bar code scanner or other means), a means for storing the medical data (such as a computer memory), a processor for applying calculations, rules and comparisons on the data, and a means of displaying or storing the results (in the shape of for instance the medication chart) such as a computer monitor, a printer, and/or an external computer memory.

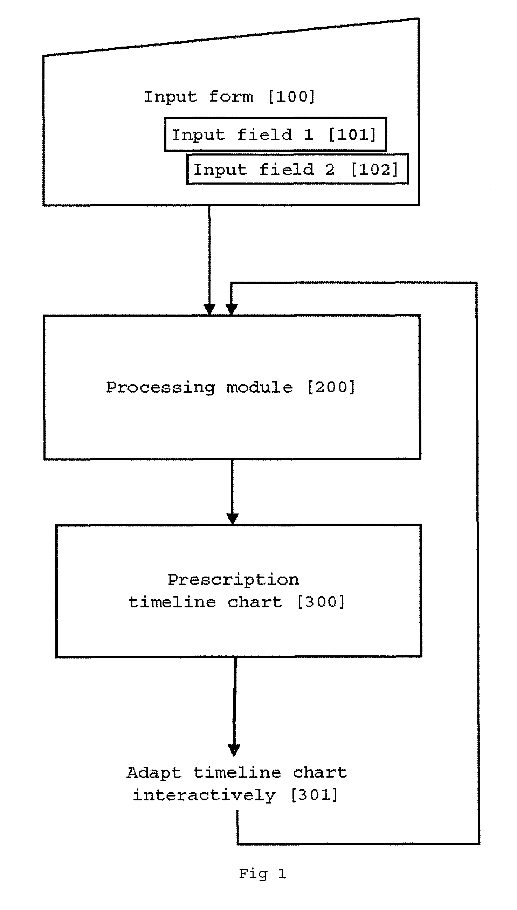

Fig. 1 represents a block diagram illustrating the method of creating the medical prescription chart as described in an embodiment of the invention. As a first step, the user input is collected by means of an input form [100] which is presented to the user. A set of medical information is requested from the user by presenting an input form allowing the different elements of the medication prescription to be entered in separate input fields [101, 102]. The set of medical information makes up the medication prescription, the parameters to be supplied may vary depending on the chosen medication regime for the particular drug to be administered. When all accessible input fields on the input form have been supplied with input, a valid prescription is the result of this input.

Every element of entered user input through an input field [101, 102] is passed immediately to the processing module [200] which comprises a predefined set of instructions that could be used to interpret the medical information. Alternatively, the analysis module could include a look up table based on which the medical information is interpreted.

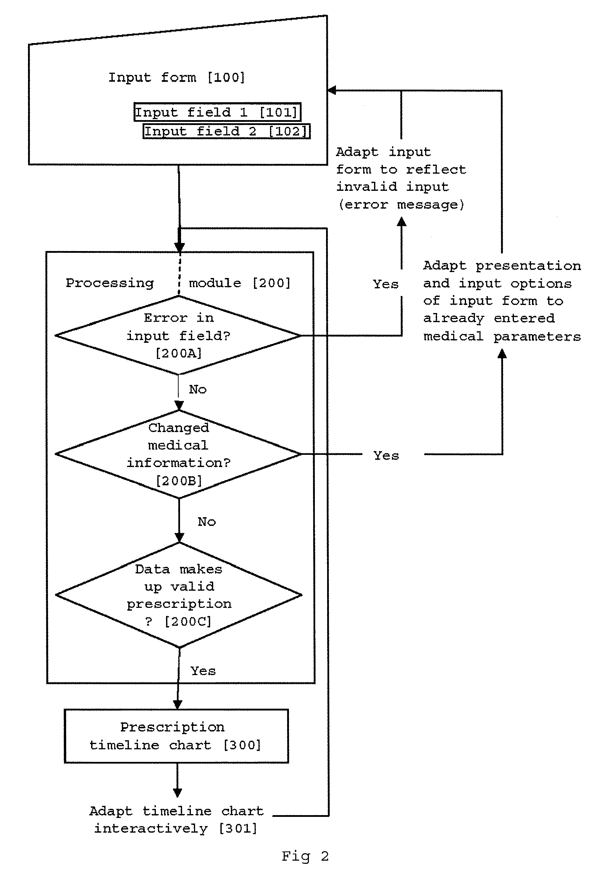

Fig 2. represents a block diagram illustrating the method of claim 2, where the processing module [200] interprets and processes the data by evaluating potentially 3 different things:
1) [200A] - the validity of the entered input for the individual input field [101] and [102] where the data just has been entered. In one embodiment of this invention the evaluation module [200] checks for instance whether the entered values fall within the expected ranges for the individual input fields. Another embodiment may implement a verification of the input for invalid characters or unexpected input types. In yet another embodiment, the input data (which is a formula expression, such as "2/2/2/2" or "5/4/1" or "1/1/1") is entered as a formula representing a sequence of dose administrations, which needs to be analysed and decomposed into a set of individual medical data.
2) [200B] - whether the changed or added input stored in the memory (through entry of data in the input field [101],[102], or by changes made on the interactive timeline chart [300]) has an impact on the medical information of the prescription, and thus on the layout and/or the contents of the input form [100]. As an example, a change of the dosage application type will always have an impact on the layout of the input form [100]. As another example, removing a dosage event from the interactive medication timeline will always result in a change of the value shown in the dosage input field.
3) [200C] - the completeness of the set of medical information making up a valid medication prescription. The evaluation module [200] will decide whether the set of medical information represents unambiguously a valid prescription.

In the first case above [200A], the evaluation module [200] will decide whether to accept the input or to adapt the input form to reflect the invalid input entered in an input field [200A]. In one embodiment, the input form is changed by showing an error message on the input field [101, 102] expressing the unsatisfied input condition in relation with the particular input field. In the same case [200A], but in a different embodiment, the input form is changed by showing a suggestion to correct the incorrect data entry made in the input field. Another embodiment may, the input field [200A] may show up in a different color (for instance red) to suggest that an invalid entry was made in the concerned input field.

Subsequently to the above step [200A], the evaluation module [200] may decide to adapt the input form to reflect the selection of a different dosage application type (continuous or continuous) [200B]. The change in the medication prescription of the dosage application type has an immediate impact on the layout of the input form. As an example, in one embodiment, if the selected dosage application type is changed to "Continuous", the input fields for entering/defining the dosage timing and distribution over the day are no longer relevant for the prescription, and will be omitted from the input form.

Subsequently to the above step [200B], the evaluation module [200] may decide to adapt the prescription timeline chart [300] to reflect the validated prescription in step [200C]. Every time the condition of [200C] is met, the prescription timeline chart [300] will be updated.

The prescription timeline [300] is interactive, meaning that the different elements or icons representing a dosage event can be moved or dragged, expanded or shrunken, duplicated or deleted, depending on the characteristics of the icons themselves. Different dosage events are represented on the dosage timeline chart as different icons. Discrete dosage events (having no duration) are represented as an icon showing the dosage form (in the shape of a pill, injection, drop,...), whereas dosage events with a certain duration are represented on the prescription timeline as a bar indicating the time period of the application.

The discrete dosage event icons can be moved on the timeline to alter their intended dosage timing. The discrete dosage events can also be duplicated or removed from the timeline in an interactive way (i.e. by performing an operation on the icon by means of the available input devices of the computer implementation, which are typically: a computer mouse, a touch screen, or keyboard keystrokes).

Dosage events with a duration are shown as a bar on the timeline; the length of the bar representing the duration of the event. The said bar can be removed or duplicated, but also can be moved (to change the starting time of the event), stretched or shrunken (make the duration longer or shorter respectively) in order to alter the medication prescription interactively.

Any changes to the prescription timeline [300] are also coupled back to the input form [100] in order to make sure that the input form reflects the medication prescription correctly.

In an embodiment, the icon representing the start-time of the medication prescription for the patient is represented with an icon on the same prescription time-line depicting a clock or watch.

## Claims

1. A computer-implemented method for entering a medication prescription for a patient, comprising the steps of:
- entering medical information related to the medication prescription in an input module, and storing said information in a memory,
- interpreting and processing the stored medical information in a processing module,
- generating a medical prescription time-line chart **characterized in that**
- when the user interacts with graphical elements on the time-line chart, the medical information is updated in memory.

2. The method of claim 1 wherein the processing module is triggered to interpret and process the data in memory, when the medical information is updated in memory, said processing comprising at least one of:
- a validation of the data entered in the individual input elements of the input module
- an evaluation if the changed medical data has an impact on the input module layout and/or content
- an evaluation if the changed medical data comprise a complete valid prescription,
wherein the presentation and/or input options of the input module are adapted by means of adding or removing fields for entering medical information.

3. The method of claim 2 wherein the processing module selects an appropriate graphical element depending on a type of dosage form in the prescription.

4. The method of claim 3 wherein an appropriate graphical element for a discrete dosage form is an icon depicting the dosage form, and wherein an appropriate graphical element for a dosage form with a duration is a bar with a length representing a dosage duration.
